# EUROPEAN PATENT APPLICATION

(11) **EP 2 109 056 A2**
(43) Date of publication of application: **14.10.2009**
(21) Application number: 09157483.0
(22) Date of filing: 07.04.2009
(51) Int. Cl.: G06F 19/00

(54) **System and method for dynamic drug interaction analysis and reporting**

(30) Priority: 08.04.2008 US 43324 P
(71) Applicant: The Quantum Group, Inc., Wellington FL 33414 (US)
(72) Inventor: Martinez, Pedro, Boca Raton, FL 33431 (US); Guillama, Noel, Wellington, FL 33414 (US)
(74) Representative: Eisenführ, Speiser & Partner

(57) **Abstract**

A system for capturing, synthesizing, analyzing, and reporting drug performance data. The system can comprise one or more electronic data processors and a data communications interface connected with the one or more processors for accessing a plurality of databases containing drug analysis and best-practice guidelines, patient data, and demographic data. The system can also include a situational modeler configured to execute on the electronic data processor in order to integrate the best-practice guidelines, patient data, and demographic data, create best-practice templates for analysis, based upon the integration of the guidelines, patient data, and demographic data, analyze the best-practice guidelines, patient data, and demographic data, and generate a series of reports and analyses to determine drug performance, based upon the analysis.

## Description

The present invention is related to the fields of data analysis, processing, and reporting, and more particularly, to computer-based systems and methods for integrating, analyzing, and processing data from a variety of sources so as to generate drug performance reports and analyses.

A great challenge for both pharmaceutical companies and healthcare providers is how to provide the measurement and reporting of drug performance and interactions, while at the same time providing a high level of statistical significance and in-depth variable analysis. Additionally, it has become increasingly more important to be able to detect and predict trends, side effects, drug interactions, and potential issues with respect to various drugs before they become widespread in the community. Knowing such trends and potential issues before they become widespread would be very useful in alerting physicians to problematic drugs, limiting the number of people affected by such drugs, enabling pharmaceutical companies to begin improving drugs sooner, and substantially lowering health-related costs.

As a result, there is a need for more effective efficient means of analyzing and reporting drug performance. Furthermore, there is a need for effective and efficient methods and systems to access, integrate, and analyze health-related data and to generate drug performance reports.

The present invention is directed to systems and methods for capturing, synthesizing, integrating, and analyzing health-related data from a variety of data sources, and using the integration and analysis to generate reports containing drug performance data and analyses.

One embodiment of the invention is a system for capturing, synthesizing, analyzing, and reporting drug performance data. The system can comprise one or more electronic data processors and a data communications interface connected with the processor(s) for accessing a plurality of databases containing drug analysis and best-practice guidelines, patient data, and demographic data. The system can also include a situational modeler configured to execute on the one or more electronic data processors in order to integrate the best-practice guidelines, patient data, and demographic data, create best-practice templates for analysis, based upon the integration of the guidelines, patient data, and demographic data, analyze the best-practice guidelines, patient data, and demographic data, and generate a series of reports and analyses to determine drug performance, based upon the analysis.

Another embodiment of the invention is a computer-based method for capturing, synthesizing, analyzing, and reporting drug performance data. The method can include accessing a plurality of databases containing drug analysis and best-practice guidelines, patient data, and demographic data. The method can also include integrating the best-practice guidelines, patient data, and demographic data and creating best-practice templates for analysis which are based on the integration of the guidelines, patient data, and demographic data. The method can further include analyzing the best-practice guidelines, patient data, and demographic data and generating a series of reports and analyses to determine drug performance which is based on the analysis.

Yet another embodiment of the invention is a computer-readable medium which contains computer-readable code that when loaded on a computer causes the computer to access a plurality of databases containing drug analysis and best-practice guidelines, patient data, and demographic data, to integrate the best-practice guidelines, patient data, and demographic data, to create best-practice templates for analysis, based upon the integration of the guidelines, patient data, and demographic data, to analyze the best-practice guidelines, patient data, and demographic data, and to generate a series of reports and analyses to determine drug performance, based upon the analysis.

There are shown in the drawings, embodiments which are presently preferred. It is expressly noted, however, that the invention is not limited to the precise arrangements and instrumentalities shown.
- Fig. 1: is a schematic view of a system for integrating and analyzing best practice guide-lines, patient data, and demographic data and for generating drug performance reports and analyses, according to one embodiment of the invention.
- Fig. 2: is a flowchart of steps in a method for integrating and analyzing best practice guidelines, patient data, and demographic data and for generating drug performance reports and analyses, according to another embodiment of the invention.

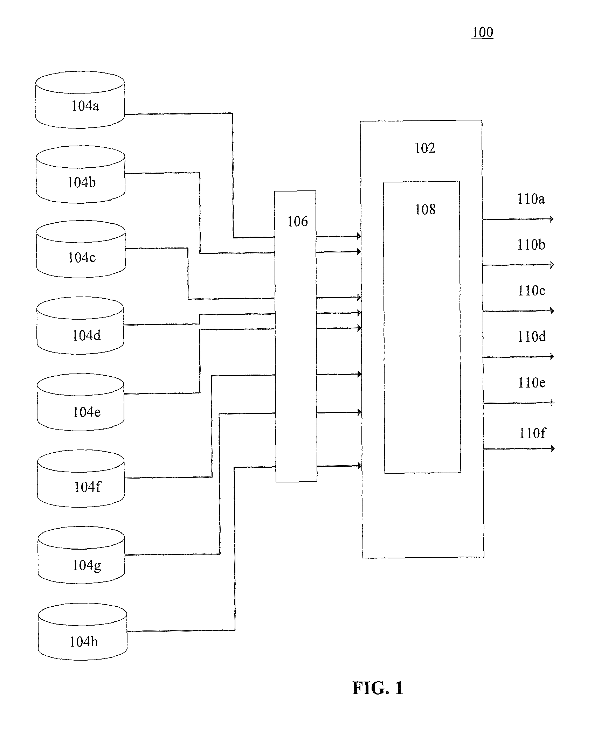

Referring initially to Fig. 1, a system 100 for capturing, synthesizing, analyzing, and reporting drug performance data, according to one embodiment of the invention, is schematically illustrated. The system includes one or more electronic data processing elements 102. The system 100 further includes a plurality of databases 104. Even though eight databases 104a-h are shown, it will be apparent to one of ordinary skill based on the description that a greater or lesser number of databases can be utilized.

The system 100 further includes a data communications interface 106 which is communicatively connected with the electronic data processing elements 102. As shown, the data communications interface 106 communicatively links each of the databases 104a-h to another component of the system 108. This component is a situational modeler 108, which, if implemented as computer-readable code, is configured to execute on the electronic data processing elements 102. Alternatively, in another embodiment, the databases 104a-h can communicatively link to the situational modeler 108 through a data communications network (not explicitly shown). The data communications network can be a local-area network (LAN), wide-area network (WAN), or the Internet.

Alternatively, the situational modeler 108 can be implemented in hardwired, dedicated circuitry for performing the operative functions described herein. In another embodiment, the situational modeler 108 can be implemented in computer-readable code configured to execute on a particular computing machine. In yet another embodiment, however, the situational modeler can be implemented in a combination of hardwired circuitry and computer-readable code.

Operatively, the data communications interface 106 accesses the plurality of databases 104a-h. As previously mentioned, the number of databases contained in the system 100 can be greater or fewer than the quantity illustrated in Fig. 1. The databases 104a-h can include best-practice guidelines data which can comprise Food and Drug Administration Guidelines, pharmaceutical guidelines, and industry reference data. Also, the databases 104a-h can include a patient database which can comprise data from sources including doctors, hospitals, other healthcare providers, and patients. Additionally, the databases 104a-h can include demographic data, including for example age-related data, gender-related data, and other information that can be useful in determining drug performance and interactions.

Operatively, the situational modeler 108 integrates the best-practice guidelines, patient data, and demographic data accessed through the data communications interface 106 into best-practice templates and formats the data. The type of data accessed by the situational modeler 108 comprises data pertaining to age, gender, weight, drug usage, profession, ethnicity, geography, and other types of relevant data. The aggregation of the data from multiple sources and patients creates statistically valid samples of various populations. Also, the situational modeler 108 analyzes the best-practice guidelines, patient data, and demographic data and generates a series of reports and analyses 110a-f to determine drug performance and interactions. Even though six reports and analyses are shown, it will be apparent to one of ordinary skill that a greater or lesser number of reports and analyses can be generated. The reports and analyses 110a-f can comprise drug usage reports, trends analysis, potential concerns reports, advanced studies reports, expert analysis of data and reports, and drug interaction analysis.

The situational modeler 108 can be configured to identify which best-practice guidelines are to be utilized with a particular set of patient data and demographic data. For example, the situational modeler 108 can select a particular pharmaceutical guideline to be utilized with a patient having a particular age, weight, and drug usage history. The situational modeler 108 can be further configured to provide monitoring of drugs and real-time alerts which can identify potential areas of concern.

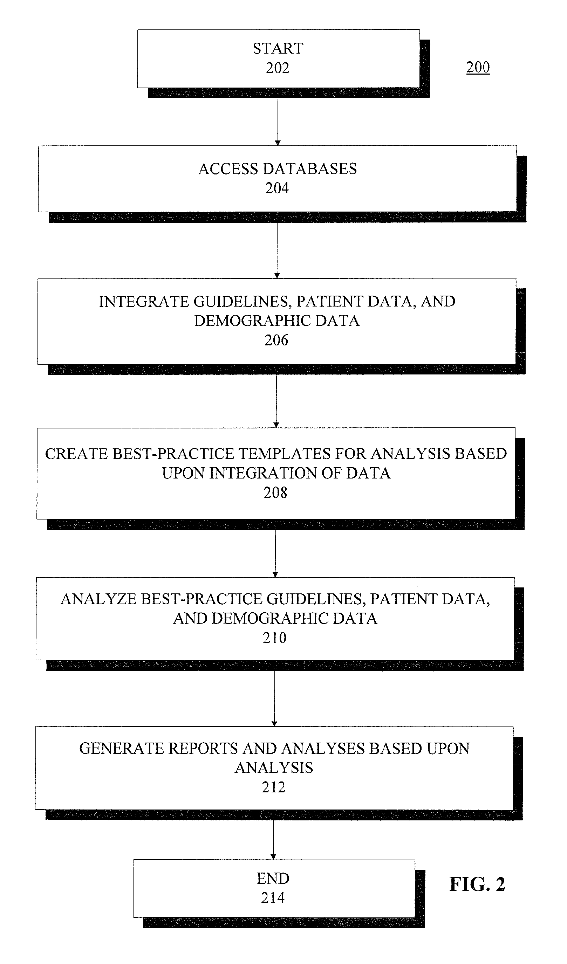

Referring now to Fig. 2, a flowchart is provided that illustrates certain method aspects of the invention. The flowchart depicts steps of a method 200 for integrating and analyzing best practice guidelines, patient data, and demographic data and generating drug performance reports and analyses. The method 200 illustratively includes, after the start step 202, accessing a plurality of databases containing drug analysis and best-practice guidelines, patient data, and demographic data at step 204. The method 200 also includes integrating the best-practice guidelines, patient data, and demographic data at step 206. Additionally, the method 200 includes at step 208 creating best-practice templates for analysis, based upon the integration of the guidelines, patient data, and demographic data. The method 200 also includes analyzing the best-practice guidelines, patient data, and demographic data at step 210. The method 200 further includes at step 212 generating a series of reports and analyses to determine drug performance, based upon the analysis. The method 200 illustratively concludes at step 214.

The method 200 can also include identifying which best-practice guidelines are to be used with a particular set of patient data and demographic data through the use of a situational modeler. For example, the identification can involve selecting a particular pharmaceutical guideline to be utilized with a patient having a particular age, weight, and drug usage history.

According to another embodiment, the method 200 can further include providing monitoring of drugs and real-time alerts which can identify potential areas of concern.

The invention, as already mentioned, can be realized in hardware, software, or a combination of hardware and software. The invention can be realized in a centralized fashion in one computer system, or in a distributed fashion where different elements are spread across several interconnected computer systems. Any type of computer system or other apparatus adapted for carrying out the methods described herein is suitable. A typical combination of hardware and software can be a general purpose computer system with a computer program that, when being loaded and executed, controls the computer system such that it carries out the methods described herein.

The invention, as already mentioned, can be embedded in a computer program product, such as magnetic tape, an optically readable disk, or other computer-readable medium for storing electronic data. The computer program product can comprise computer-readable code, (defining a computer program) which when loaded in a computer or computer system causes the computer or computer system to carry out the different methods described herein. Computer program in the present context means any expression, in any language, code or notation, of a set of instructions intended to cause a system having an information processing capability to perform a particular function either directly or after either or both of the following: a) conversion to another language, code or notation; b) reproduction in a different material form.

The preceding description of preferred embodiments of the invention has been presented for the purposes of illustration. The description provided is not intended to limit the invention to the particular forms disclosed or described. Modifications and variations will be readily apparent from the preceding description. As a result, it is intended that the scope of the invention not be limited by the detailed description provided herein.

## Claims

1. A computer-based method for capturing, synthesizing, analyzing, and reporting drug performance data, the method comprising:
accessing a plurality of databases containing drug analysis and best-practice guidelines, patient data, and demographic data;
integrating the best-practice guidelines, patient data, and demographic data;
creating best-practice templates for analysis, based upon the integration of the guidelines, patient data, and demographic data;
analyzing the best-practice guidelines, patient data, and demographic data; and
generating a series of reports and analyses to determine drug performance, based upon the analysis.

2. The method of Claim 1, wherein the plurality of databases comprise at least one of a best-practice guidelines database, a patient database, and a demographic database; and wherein the step of accessing comprises retrieving at least one of best-practice guidelines from the best-practice guidelines database, patient data from the patient database, and demographic data from the demographic database.

3. The method of Claim 1, wherein said patient data are selected from at least one among doctor-patient data, hospital records, related care provider data and patient input, or
wherein said best practice guidelines are selected from at least one among Food and Drug Administration guidelines, pharmaceutical guidelines, and industry reference data, or
wherein said reports and analyses comprise at least one of a drug usage report, trends analysis, potential concerns reports, advanced studies reports, expert analysis of data and reports, and drug interaction analysis reports.

4. The method of Claim 1, wherein the step of integrating said best-practice guidelines, said patient data, and said demographic data comprises using a situational modeler, wherein said situational modeler identifies which best-practice guidelines are to be used with a particular set of patient data and demographic data,
optionally
further comprising providing monitoring of drugs and
real-time alerts identifying potential areas of concern.

5. A computer-based system for capturing, synthesizing, analyzing, and reporting drug performance data, the system comprising:
at least one electronic data processor;
a data communications interface connected with said processor for accessing a plurality of databases containing drug analysis and best-practice guidelines, patient data, and demographic data;
a situational modeler configured to execute on said electronic data processor, in which said situational modeler is configured to:
integrate the best-practice guidelines, patient data, and demographic data,
create best-practice templates for analysis, based upon the integration of the guidelines, patient data, and demographic data,
analyze the best-practice guidelines, patient data, and demographic data, and
generate a series of reports and analyses to determine drug performance, based upon the analysis.

6. The system of Claim 5, wherein the plurality of databases comprise at least one of a best-practice guidelines database, a patient database, and a demographic database; and wherein accessing comprises retrieving at least one of best-practice guidelines from the best-practice guidelines database, patient data from the patient database, and demographic data from the demographic database.

7. The system of Claim 5, wherein said patient data are selected from at least one among doctor-patient data, hospital records, related care providers and patient input, or
wherein said best practice guidelines are selected from at
least one among Food and Drug Administration guidelines, pharmaceutical guidelines, and industry reference data, or
wherein said reports and analyses comprise at least one
of a drug usage report, trends analysis, potential concerns reports, advanced studies reports, expert analysis of data and reports, and drug interaction analysis reports.

8. The system of Claim 5, wherein said situational modeler identifies which best-practice guidelines are to be used with a particular set of patient data and demographic data, optionally
further comprising providing monitoring of drugs and
real-time alerts identifying potential areas of concern.

9. A computer-readable storage medium having stored therein computer-readable instructions, which, when loaded in and executed by a computer causes the computer to perform the steps of:
accessing a plurality of databases containing drug analysis and best-practice guidelines, patient data, and demographic data;
integrating the best-practice guidelines, patient data, and demographic data;
creating best-practice templates for analysis, based upon the integration of the guidelines, patient data, and demographic data;
analyzing the best-practice guidelines, patient data, and demographic data; and
generating a series of reports and analyses to determine drug performance, based upon the analysis.

10. The computer-readable storage medium of Claim 9, wherein the plurality of databases comprise at least one of a best-practice guidelines database, a patient database, and a demographic database; and wherein the step of accessing comprises retrieving at least one of best-practice guidelines from the best-practice guidelines database, patient data from the patient database, and demographic data from the demographic database.

11. The computer-readable storage medium of Claim 9, wherein said patient data are selected from at least one among doctor-patient data, hospital records, related care providers and patient input, or
wherein said best practice
guidelines are selected from at least one among Food and Drug Administration guidelines, pharmaceutical guidelines, and industry reference data, or
wherein said reports and analyses comprise at least one of a drug usage report, trends analysis, potential concerns reports, advanced studies reports, expert analysis of data and reports, and drug interaction analysis reports.

12. The computer-readable storage medium of Claim 9, further comprising code for causing the computer to provide monitoring of drugs and real-time alerts identifying potential areas of concern.
